# EUROPEAN PATENT SPECIFICATION

(11) **EP 1 401 516 B1**
(45) Date of publication and mention of the grant of the patent: **07.09.2005**
(21) Application number: 02730518.4
(22) Date of filing: 18.06.2002
(51) Int. Cl.: A61M 5/00

(54) **A DEVICE FOR CONTROLLING THE ADMINISTRATION OF A MATERIAL SUCH AS MEDICINE OR BLOOD**
VORRICHTUNG ZUR STEUERUNG DER ABGABE EINES MATERIALS, Z.B. EIN MEDIKAMENT ODER BLUT
DISPOSITIFS D'ADMINISTRATION DE SUBSTANCE

(30) Priority: 30.06.2001 GB 0116036
(43) Date of publication of application: 31.03.2004
(73) Proprietor: Maddocks, John Leyshon, Sheffield S10 3EJ (GB)
(72) Inventor: Maddocks, John Leyshon, Sheffield S10 3EJ (GB)
(74) Representative: Swindell & Pearson
(86) International application number: PCT/GB2002/002776
(87) International publication number: WO 2003/004077

(56) References cited:
- EP-A- 1 092 447
- WO-A-01/23017
- US-A- 4 592 746
- US-A- 5 925 032

## Description

The present invention relates to devices for administering material and in particular but not exclusively to devices for use in the administration of material such as medicine or other therapeutic material to a body, such as the human or animal body.

The administration of medicine or other therapeutic material, such as blood, to the human or animal body requires care, particularly to ensure that the material is appropriately administered to the body.

Sometimes inappropriate administration of material can lead to serious difficulties and complications to the body to which it is administered. For example, if the route of administration of the material is inappropriate this can cause difficulties, and indeed can be fatal. For example, the cytotoxic drug Vincristine requires to be injected intravenously, but there have been instances when this drug has been accidentally injected into the spine in error with often fatal consequences. Other examples include the administration of local anaesthetic Bupivacaine which when injected intravenously instead of in the epidura can be fatal. Similar injection errors have occurred with other drugs such as adrenaline, potassium chloride and neuro-muscular blocking agents.

Many approaches have been considered to address these issues, which arise predominantly because drugs are administered by humans and humans are prone to human error. Increases in complexity of procedures and protocols for drug administration has been one approach, but these can sometimes in themselves establish environments in which errors are likely to occur.

The administration of materials to other bodies or systems can often also require extreme care to ensure appropriate administration. For example, the addition of chemicals to water systems, constituents to foodstuffs and drinks etc. can sometimes require very careful administration to prevent potentially hazardous or otherwise undesirable situations arising.

According to the present invention there is provided a device as defined in claim 1.

The input means may comprise mechanical means, such as manually operable members, for example rotatable lock tumblers. Alternatively or in addition the lock means may be electronically operable.

The device may comprise comparison means to compare information inputted into the device with predetermined information accessible to the comparison means and to enable the device to be moved to the second condition when the inputted information is deemed appropriate by the comparison means. The comparison means may comprise a memory to hold information. The memory may hold predetermined information, such as information relating to the material to be administered, the body, such as a patient, to which the material is to be administered. Alternatively or in addition some or all of the predetermined information may be held remotely from the device to be accessible thereby.

Preferably the indicating means comprises a sign such as one or more letter(s) and/or symbol(s) advising an observer of the correct administration of the material using the apparatus. Preferably the indicating means notifies an observer, in particular a user of the device, of the correct route of administration of material using the apparatus.

Preferably the indicating means advises, either directly or indirectly, the user of the device of the appropriate manipulation of the device required to unlock the lock means.

The indicating means may comprise a string of symbols relating to the administration of material with the apparatus, which string of symbols when appropriately aligned on the input means enable, release of the lock means.

The input means may comprise means to send electronic signals to unlock the lock means, and may comprise buttons, keys or similar means. The device may comprise an electronic display, which may provide the indicating means and/or display data inputted into the device.

The device may comprise transmitter means to send signals to remote data processing apparatus, such as a computer, server, Internet apparatus or the like. The device may comprise receiver means operator to receive signals, for example signals sent from a computer, server or such means, which signals may be processed by processing means provided in the device.

The device may comprise a body locatable, in use, around a section or part of administration apparatus. The device is preferably locatable in use on the plunger of a syringe used to administer material. Alternatively or in addition the device may be locatable in use on a material supply line, such as an ivi line.

The body preferably comprises a first part movably attached to a second part with the lock means operable to secure the parts around the aspects in the first condition. The parts may be pivotally attached, with the lock means preferably acting to clamp the parts around the apparatus when in the first condition.

Preferably the device prevents or restricts movement of the plunger into the syringe when in the first condition on the syringe whereby to prevent administration of the material in the syringe.

The device may further comprise a remote control unit, such as a computer, palm top computer, telephone, mobile telephone, pager or similar such device to enable remote control of the body.

The invention also provides apparatus for administering material to a body, the apparatus comprising a device as described in any of the preceding paragraphs.

Preferred embodiments of the present invention will now be described by way of example only, with reference to the accompanying drawings in which:-
Fig. 1 is a diagrammatic exploded view of a device according to the present invention;
Fig. 2 is a reduced scale diagrammatic representation of the device of Fig. 1 in use on a syringe; and
Fig. 3 is a view similar to Fig. 2 showing a device according to an alternative embodiment of the present invention.

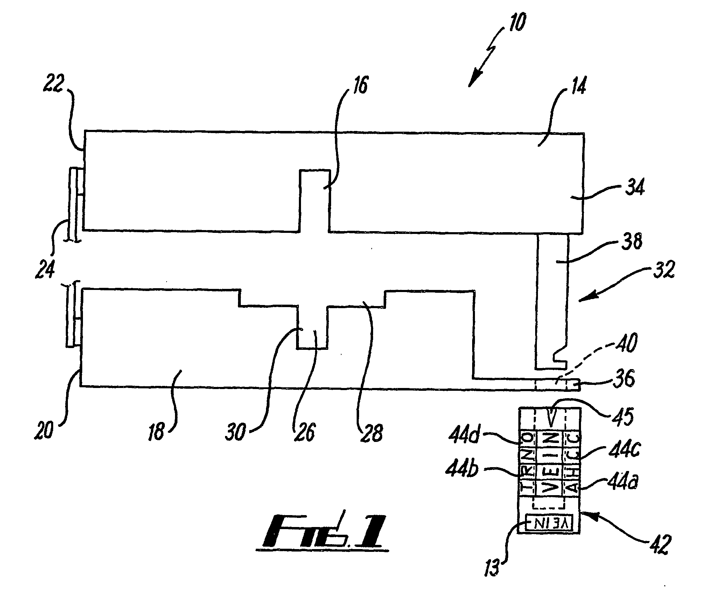

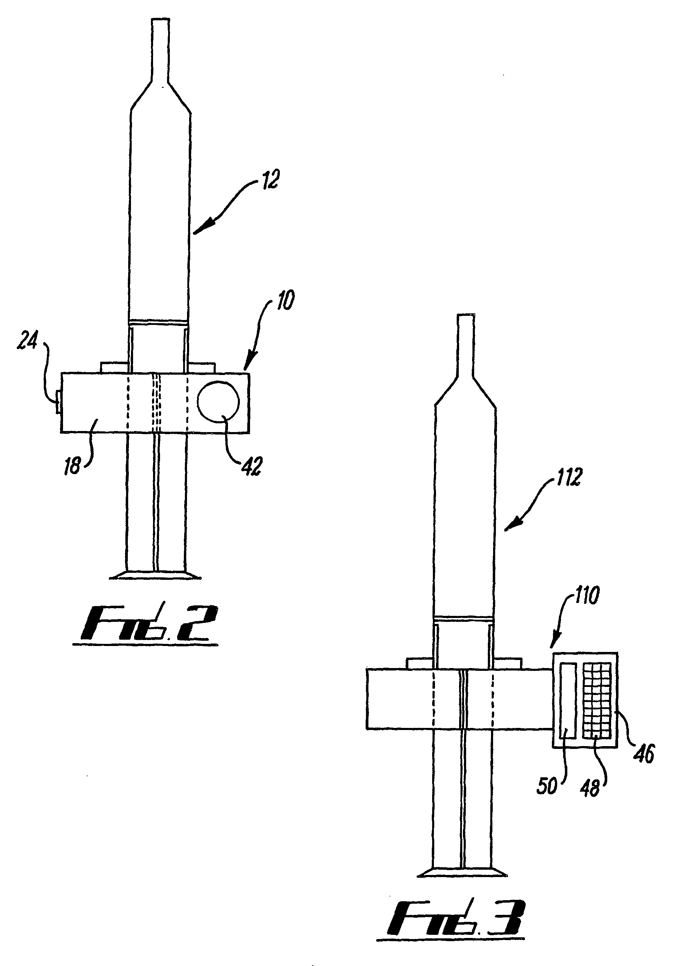

Referring to the drawings, there is provided a device 10 for use in the administration of material to a body (not shown), the device 10 being located for use upon administration apparatus, shown in the form of a syringe 12 (Fig. 2) to be selectively moveable between a first condition in which the administration of material from the apparatus 12 is prevented and a second condition which allows material to be dispensed from the apparatus 12 and comprising means 13 indicating appropriate administration of material from the apparatus 12.

In more detail, the device 10 comprises a first part 14 of generally rectangular cross-section and comprising a recess 16 on one side generally mid-way along its length.

A second part 18 is pivotally attached at one end 20 to an end 22 of the part 14 by a linkage 24 (which is shown broken in Fig. 1 but which would provide pivotal connection between the two parts 14 and 18).

A re-entrant portion 26 is provided generally mid-way along the length of a side of the second part 18. The re-entrant portion 26 comprises a shallow portion 28 generally from the centre of which extends a narrower portion 30 into the body of the part 18. Between the recess 16 and re-entrant portion 26 is defined a generally cruciform section to receive and therefore allow the device 10 to locate around, the plunger of syringes which conventionally have cruciform cross-sections. It will however be appreciated that the shape of the section defined between the parts 14, 18 can be varied and determined according to the shape of the apparatus or part thereof around which the device 10 is to locate in use.

Lock means 32 is provided to be operable between the respective other ends 34, 36 of the part 14, 18. The lock means 32 comprises a shaft 38 extending from the end 34 to be locatable through an opening 40 in the end 36 of the other part 18 to receive a tumbler mechanism 42 of generally known design. The lock means 32 itself is of generally known design. The tumbler mechanism 42 comprises a series of four finger moveable tumblers 44 a, b, c, d around each of which is provided a series of letters of the alphabet.

The indicating means 13 comprises a sticker bearing a word, in this example 'vein' indicating the appropriate route for administration of material.

In use, the device can be used in hospitals and other institutions where drugs and other therapeutic material are administered to humans, or indeed animals.

Particularly with regard to drugs, these are often dispensed by pharmacists or similar practitioners who load the appropriate material and dosage into the apparatus that will be used to administer it, in most cases a syringe. This stage is usually double checked by a second pharmacist.

Once the syringe has been loaded with material, the pharmacist applies the sticker 13 on the device 10, preferably in the vicinity of the tumbling mechanism 42. In this particular example the material is to be administered intravenously, and therefore an appropriate indication to the person who is to, administer the material (which is not the pharmacist) is 'vein' and the sticker bears this word.

It will of course be appreciated that any appropriate indication, sign or symbol could be used to indicate appropriate administration although it is preferable that the meaning of any indication used renders it obvious to personnel who will be administering the material what the correct administration is. Another example could be the code 'thec' to indication intrathecal administration.

The device 10 is then securely located around the plunger of the syringe 12 with the plunger 12 located within the opening defined between the recess 16 and re-entrant portion 26, and the lock means 32 used to secure the device 10 in this first position. The precise location of the device 10 on the plunger 12 is determined according to the degree of movement of the plunger in the cylinder of the syringe that is to be prevented.

The syringe 12 and the material therein are now secure against inappropriate administration. The device 10 can only be removed from the first condition around the syringe 12 when the lock means 32 is released, which in this example require the tumblers 44a, b, c, d to be appropriately manipulated to align the letters of the four tumbler 14a, b, c and d to spell out, at the appropriate location, the word 'vein'. A mark 45 adjacent the tumbler indicates the location of alignment.

It will be seen that in use, personnel to administer material from apparatus 12 secured with the device 10 have to consciously insert the correct route of administration to unlock the device 10 from the first condition and thereby allow the device 10 to be moved from around the plunger and thus allow administration of the material from the syringe. The requirement for this step ensures that personnel administering the material consciously note the route of administration before administering the material which significantly reduces the possibility of inappropriate administration.

Fig. 3 shows an alternative embodiment of a device 110, similar to device 10 but which comprises an electronically operable lock means 46 which comprises an array of buttons or keys 48 in which data can be entered to release the lock means 46 and a display 50 which is operable as indicating means to display the appropriate administration, and which may also display other data, such as the entries being made by a user to release the device 110.

The lock means 46 may be of any appropriate design, and the device 110 functions in essentially the same way as the device 10 described above.

Additional features may be incorporated with electronic lock means 46. For example, the device 110 may be able to display and/or receive further data such as patient number, patients history, details of the material being administered, drug contraindications, drug interactions, dosage and the like. However, it is important that the device 110 displays the appropriate administration and requires the personnel to administer material to consciously note and input appropriate data to release the lock means 46 and thereby enable administration.

It will be appreciated that the indicating means could be used to indicate other aspects of administration, other than or in addition to the route of administration. It could for example identify the patient to whom material is to be administered.

It will further be appreciated that particularly with the electronic device 110 data could be transmitted to remote data processing equipment, such as a computer server Internet apparatus and the like, which could record information from and send information to the device, including date and time of administration, administrator, etc. The device can also comprise receiver means to receive and process data signals, for example signals sent by a remote computer server or the like.

The device may also comprise a remote controller, such as a hand held device like a palm top computer, telephone, mobile telephone, pager, computer or similar such device to enable remote control of the body on the administration apparatus.

The device may be operable using infra red and/or radio frequency signals.

In a further embodiment of the invention the device comprises comparison means, such as a computer processor unit, which is operable to compare information inputted into the device by a user with predetermined information accessible to the comparison means. The comparison means controls movement of the device to the second condition, only allowing the device to assume the second condition when information inputted into the device is appropriate and compares favourably with the predetermined information. The predetermined information can be stored in a memory of the device and/or remotely, for example in (a) computer database(s) or the like. The predetermined information can relate to any aspect that is relevant to the appropriate administration of material from the apparatus, such as information relating to the body to which material is to be administered including patient information, drug therapy, allergies, information relating to the material to be administered.

In use, the device of the embodiment could be regarded by a user as effectively the body to which material is to be administered. In this way, a user inputs information into the device as if it were administering material. If the inputted information is considered correct and appropriate by the comparison means, the means moves or enables the device to be moved to the second condition where the apparatus on which the device is operable can administer material therefrom.

It is also within the scope of the present invention that a plurality of devices could be used on a single administration apparatus providing still further security against inappropriate administration.

The invention can also be used in the administration of material to any other body, system, arrangement or composition, particularly where material has to be carefully administered. For example the invention could be used in the administration of one or more chemicals into fluid systems such as water systems and water treatment systems, the administration of ingredients into beverages and foodstuffs.

## Claims

1. A device for use in controlling the administration of material by administration apparatus, the device comprising a body locatable around a section or part of administration apparatus, said body being selectively movable between a first condition in which the administration of material from the apparatus is prevented and a second condition which allows material to be dispensed from the apparatus, and the device further including means indicating appropriate administration of material from the apparatus, and lock means operable to selectively releasably secure the body in the first condition, wherein the lock means is configured to be operable to release the body and allow the body to move to the second condition only on inputting to the lock means a combination of symbols relating to the administration of the material.

2. A device according to daim 1, wherein the combination of symbols and the indicating means correspond to one another.

3. A device according to claim 1 or 2, wherein the lock means comprises input means for inputting to the lock means said combination of symbols.

4. A device according to claim 3, wherein the input means comprises a plurality of input members for inputting the combination of symbols to the lock means.

5. A device according to claim 3 or 4, in which the input means comprises mechanical means.

6. A device according to claim 5, in which the input means comprises rotatable lock tumblers.

7. A device according to claim 3 or 4, in which the lock means may be electronically operable.

8. A device according to claim 7, in which the device comprises comparison means to compare information inputted into the device with predetermined information accessible to the comparison means and to enable the device to be moved to the second condition when the inputted information is deemed appropriate by the comparison means.

9. A device according to claim 8, in which the comparison means comprises a memory to hold information.

10. A device according to claim 9, in which the memory may hold predetermined information.

11. A device according to claim 10, in which the memory holds information relating to the material to be administered, the body, such as a patient, to which the material is to be administered.

12. A device according to any of claims 8 to 11, in which some or all of the predetermined information is held remotely from the device to be accessible thereby.

13. A device according to any of claims 2 to 12, wherein the indicating means comprises a sign such as one or more letter(s) and/or symbol(s) advising an observer of the correct administration of the material using the apparatus.

14. A device according to claim 13, in which the indicating means notifies an observer, in particular a user of the device, of the correct route of administration of material using the apparatus.

15. A device according to claim 13 or 14, in which the indicating means advises, either directly or indirectly, the user of the device of the appropriate manipulation of the device required to unlock the lock means.

16. A device according to claim 15, in which the indicating means comprises a string of symbols relating to the administration of material with the apparatus, which string of symbols when appropriately aligned on the input means enable release of the lock means.

17. A device according to any of claims 7 to 16, in which the input means comprises means to send electronic signals to unlock the lock means.

18. A device according to any claims 3 to 17, in which the input means comprises buttons, keys or similar means.

19. A device according to any preceding claim, in which the device comprises an electronic display.

20. A device according to claim 19, in which the display provides the indicating means and/or display data inputted into the device.

21. A device according to any of claims 7 to 20, in which the device comprises transmitter means to send signals to remote data processing apparatus, such as a computer, server, Internet apparatus or the like.

22. A device according to any of claims 7 to 21, in which the device comprises receiver means operative to receive signals, for example signals sent from a computer, server or such means.

23. A device according to claim 22, in which the device comprises processing means to process received signals.

24. A device according to any preceding claim, in which the device is locatable in use on the plunger of a syringe used to administer material.

25. A device according to any of claims 1 to 23, in which the device is locatable in use on a material supply line, such as an ivi line.

26. A device according to any preceding claim, in which the body comprises a first part movably attached to a second part with the lock means operable to secure the parts around the apparatus in the first condition.

27. A device according to claim 26, in which the parts are pivotally attached.

28. A device according to claim 26 or 27, in which the lock means acts to clamp the parts around the apparatus when in the first condition.

29. A device according to claim 24, in which the device prevents or restricts movement of the plunger into the syringe when in the first condition on the syringe whereby to prevent administration of the material in the syringe.

30. A device according to any of claims 7 to 29, in which the device comprises a remote control unit, such as a computer, palm top computer, telephone, mobile telephone, pager or similar such device to enable remote control of the body.

31. An assembly for administering material to a body, comprising administration apparatus and a device as claimed in any of the preceding claims.

## Patentansprüche

1. Vorrichtung zur Verwendung beim Steuern der Verabreichung von Material durch ein Verabreichungsgerät, wobei die Vorrichtung einen Körper aufweist, der um einen Abschnitt oder einen Teil eines Verabreichungsgerätes anordenbar ist und wobei der Körper zwischen einem ersten Zustand, bei dem die Verabreichung des Materials aus dem Gerät verhindert wird, und einem zweiten Zustand, der die Abgabe von Material aus dem Gerät ermöglicht, selektiv bewegbar ist, und wobei die Vorrichtung außerdem ein Mittel enthält, das die richtige Verabreichung von Material aus dem Gerät anzeigt, sowie ein verriegelungsmittel enthält, das betätigbar ist, um den Körper in dem ersten Zustand selektiv lösbar zu befestigen, wobei das Verriegelungsmittel so konfiguriert ist, dass es betätigbar ist, um den Körper zu lösen und dem Körper zu ermöglichen, sich in den zweiten Zustand zu bewegen, nachdem man in das Verriegelungsmittel einfach eine Kombination von Symbolen eingibt, die sich auf die Verabreichung des Materials beziehen.

2. Vorrichtung nach Anspruch 1, **dadurch gekennzeichnet, dass** die Kombination der Symbole und die Anzeigemittel einander entsprechen.

3. Vorrichtung nach Anspruch 1 oder 2, **dadurch gekennzeichnet, dass** das Verriegelungsmittel ein Eingabemittel aufweist, um in das Verriegelungsmittel die Kombination der Symbole einzugeben.

4. Vorrichtung nach Anspruch 3, **dadurch gekennzeichnet, dass** das Eingabemittel eine Vielzahl von Eingabegliedern aufweist, um die Kombination von Symbolen in das Verriegelungsmittel einzugeben.

5. Vorrichtung nach Anspruch 3 oder 4, **dadurch gekennzeichnet, dass** das Eingabemittel mechanische Mittel aufweist.

6. Vorrichtung nach Anspruch 5, **dadurch gekennzeichnet, dass** das Eingabemittel rotierbare Verriegelungstrommeln bzw. Verriegelungstaumler aufweist.

7. Vorrichtung nach Anspruch 3 oder 4, **dadurch gekennzeichnet, dass** das Verriegelungsmittel elektronisch betätigbar ist.

8. Vorrichtung nach Anspruch 7, **dadurch gekennzeichnet, dass** die Vorrichtung ein Vergleichsmittel aufweist, um in die vorrichtung eingegebene Information mit dem Vergleichsmittel zugänglicher vorbestimmter Information zu vergleichen und um der Vorrichtung zu ermöglichen, in den zweiten Zustand bewegt zu werden, wenn die eingegebene Information durch die Vergleichsmittel als geeignet betrachtet wird.

9. Vorrichtung nach Anspruch 8, **dadurch gekennzeichnet, dass** das Vergleichsmittel einen Speicher zum Halten von Information aufweist.

10. Vorrichtung nach Anspruch 9, **dadurch gekennzeichnet, dass** der Speicher vorbestimmte Information halten kann.

11. Vorrichtung nach Anspruch 10, **dadurch gekennzeichnet, dass** der Speicher Information hält, die sich auf das zu verabreichende Material und den Körper, wie z.B. ein Patient, dem das Material verabreicht werden soll, bezieht.

12. Vorrichtung nach einem der Ansprüche 8 bis 11, **dadurch gekennzeichnet, dass** die gesamte vorbestimmte Information fern von der Vorrichtung gehalten wird, um **dadurch** zugänglich zu sein.

13. Vorrichtung nach einem der Ansprüche 2 bis 12, **dadurch gekennzeichnet, dass** das Anzeigemittel ein Zeichen, wie z.B. einen oder mehrere Buchstaben und/oder Symbole aufweist, die einem Beobachter die richtige Verabreichung des Materials unter Verwendung des Gerätes vorgibt.

14. Vorrichtung nach Anspruch 13, **dadurch gekennzeichnet, dass** das Anzeigemittel einem Beobachter, insbesondere einem Benutzer der Vorrichtung, die richtige Wegleitung der Verabreichung des Materials unter Verwendung des Gerätes mitteilt.

15. Gerät nach Anspruch 13 oder 14, **dadurch gekennzeichnet, dass** das Anzeigemittel entweder direkt oder indirekt dem Benutzer der Vorrichtung die richtige Handhabung der Vorrichtung vorgibt, die zum Entriegeln des Verriegelungsmittels notwendig ist.

16. Vorrichtung nach Anspruch 15, **dadurch gekennzeichnet, dass** das Anzeigemittel eine Aneinanderreihung von Symbolen aufweist, die sich auf die Verabreichung des Materials mit dem Gerät beziehen, wobei die Aneinanderreihung der Symbole bei richtiger Ausrichtung auf dem Eingabemittel die Freigabe des Verriegelungsmittels ermöglicht.

17. Vorrichtung nach einem der Ansprüche 7 bis 16, **dadurch gekennzeichnet, dass** das Eingabemittel ein Mittel zum Aussenden elektronischer Signale aufweist, um das Verriegelungsmittel zu entriegeln.

18. Vorrichtung nach einem der Ansprüche 3 bis 7, **dadurch gekennzeichnet, dass** das Eingabemittel Knöpfe, Schlüssel oder ähnliche Mittel aufweist.

19. Vorrichtung nach einem der vorhergehenden Ansprüche, **dadurch gekennzeichnet, dass** die Vorrichtung eine elektronische Anzeige aufweist.

20. Vorrichtung nach Anspruch 19, **dadurch gekennzeichnet, dass** die Anzeige die Anzeigemittel und/oder in die Vorrichtung eingegebene Anzeigedaten erzeugt.

21. Vorrichtung nach einem der Ansprüche 7 bis 20, **dadurch gekennzeichnet, dass** die Vorrichtung ein Sendermittel aufweist, um Signale zu einem entfernten Datenverarbeitungsgerät, wie z.B. einem Rechner, einem Server, einem Internet-Gerät oder dergleichen, zu senden.

22. Vorrichtung nach einem der Ansprüche 7 bis 21, **dadurch gekennzeichnet, dass** die Vorrichtung ein Empfängermittel aufweist, das zum Empfangen von Signalen, wie z.B. von einem Rechner, einem Server oder einem derartigen Mittel abgesendete Signale, betätigbar ist.

23. Vorrichtung nach Anspruch 22, **dadurch gekennzeichnet, dass** die Vorrichtung ein Verarbeitungsmittel zum Verarbeiten der empfangenen Signale aufweist.

24. Vorrichtung nach einem der vorhergehenden Ansprüche, **dadurch gekennzeichnet, dass** die Vorrichtung im Betrieb an dem Stempel einer Spritze angeordnet werden kann, die zum Verabreichen des Materials verwendet wird.

25. Vorrichtung nach einem der Ansprüche 1 bis 23, **dadurch gekennzeichnet, dass** die Vorrichtung im Betrieb an einer Material-Zufuhrleitung, wie z.B. einer IVI-Leitung, angebracht werden kann.

26. Vorrichtung nach einem der vorhergehenden Ansprüche, **dadurch gekennzeichnet, dass** der Körper einen ersten Teil aufweist, der an einem zweiten Teil beweglich befestigt ist, wobei das Verriegelungsmittel betätigbar ist, um die Teile um das Gerät in dem ersten Zustand herum zu befestigen.

27. Vorrichtung nach Anspruch 26, **dadurch gekennzeichnet, dass** die Teile schwenkbar befestigt sind.

28. Vorrichtung nach Anspruch 26 oder 27, **dadurch gekennzeichnet, dass** das Verriegelungsmittel so wirkt, dass es die Teile um das Gerät herum festklemmt, wenn es im ersten Zustand ist.

29. Vorrichtung nach Anspruch 24, **dadurch gekennzeichnet, dass** die Vorrichtung eine Bewegung des Stempels in die Spritze verhindert oder begrenzt, wenn an der Spritze der erste Zustand vorliegt, wodurch die Verabreichung des Materials in der Spritze verhindert wird.

30. Vorrichtung nach einem der Ansprüche 7 bis 29, **dadurch gekennzeichnet, dass** die Vorrichtung eine Fernsteuerungseinheit, wie z.B. einen Rechner, einen Handrechner, ein Telefon, ein Mobiltelefon, einen Pager oder dergleichen aufweist, um eine Fernsteuerung des Körpers zu ermöglichen.

31. Anordnung zum Verabreichen von Material an einen Körper, welche ein Verabreichungsgerät sowie eine in einem der vorhergehenden Ansprüche beanspruchte Vorrichtung aufweist.

## Revendications

1. Dispositif destiné à la commande de l'administration de matériau par un appareil d'administration, le dispositif comprenant un corps pouvant être positionné autour d'une section ou partie de l'appareil d'administration, ledit corps étant mobile sélectivement entre un premier état dans lequel l'administration de matériau à partir de l'appareil est bloquée, et un second état permettant la distribution de matériau à partir de l'appareil, et le dispositif comprenant en outre des moyens indiquant l'administration appropriée de matériau à partir de l'appareil, et des moyens de verrouillage utilisables pour fixer sélectivement de façon libérable le corps dans le premier état, dans lequel les moyens de verrouillage sont configurés pour être utilisables pour libérer le corps et permettre le déplacement du corps dans le second état uniquement en entrant dans les moyens de verrouillage une combinaison de symboles relatifs à l'administration du matériau.

2. Dispositif selon la revendication 1, dans lequel la combinaison de symboles et les moyens d'indication se correspondent.

3. Dispositif selon la revendication 1 ou 2, dans lequel les moyens de verrouillage comprennent des moyens d'entrée dans les moyens de verrouillage de ladite combinaison de symboles.

4. Dispositif selon la revendication 3, dans lequel les moyens d'entrée comprennent une pluralité d'éléments d'entrée de la combinaison de symboles dans les moyens de verrouillage.

5. Dispositif selon la revendication 3 ou 4, dans lequel les moyens d'entrée comprennent des moyens mécaniques.

6. Dispositif selon la revendication 5, dans lequel les moyens d'entrée comprennent des cylindres de verrouillage rotatifs.

7. Dispositif selon la revendication 3 ou 4, dans lequel les moyens de verrouillage peuvent être utilisés électroniquement.

8. Dispositif selon la revendication 7, dans lequel le dispositif comprend des moyens de comparaison pour comparer des informations entrées dans le dispositif avec des informations prédéterminées accessibles aux moyens de comparaison, et pour permettre le déplacement du dispositif jusqu'au second état lorsque les informations entrées sont considérées appropriées par les moyens de comparaison.

9. Dispositif selon la revendication 8, dans lequel les moyens de comparaison comprennent une mémoire stockant les informations.

10. Dispositif selon la revendication 9, dans lequel la mémoire peut stocker des informations prédéterminées.

11. Dispositif selon la revendication 10, dans lequel la mémoire stocke des informations relatives au matériau à administrer, au corps, tel que celui d'un patient, auquel le matériau doit être administré.

12. Dispositif selon une quelconque des revendications 8 à 11, dans lequel certaines des informations, voire toutes les informations, sont stockées à distance du dispositif afin d'être ainsi accessibles.

13. Dispositif selon une quelconque des revendications 2 à 12, dans lequel les moyens d'indication comprennent un signe, tel qu'une ou plusieurs lettres et/ou symboles, informant un observateur sur l'administration adéquate du matériau à l'aide de l'appareil.

14. Dispositif selon la revendication 13, dans lequel les moyens d'indication indiquent à un observateur, en particulier à un utilisateur du dispositif, la voie d'administration correcte du matériau à l'aide de l'appareil.

15. Dispositif selon la revendication 13 ou 14, dans lequel les moyens d'indication informent, de manière directe ou indirecte, l'utilisateur du dispositif sur la manipulation appropriée du dispositif nécessaire au déverrouillage des moyens de verrouillage.

16. Dispositif selon la revendication 15, dans lequel les moyens d'indication comprennent une chaîne de symboles relatifs à l'administration de matériau avec l'appareil, cette chaîne de symboles, une fois correctement alignée sur les moyens d'entrée, permettant la libération des moyens de verrouillage.

17. Dispositif selon une quelconque des revendications 7 à 16, dans lequel les moyens d'entrée comprennent des moyens pour envoyer des signaux électroniques destinés à déverrouiller les moyens de verrouillage.

18. Dispositif selon une quelconque des revendications 3 à 17, dans lequel les moyens d'entrée comprennent des boutons, des touches ou des moyens similaires.

19. Dispositif selon une quelconque revendication précédente, dans lequel le dispositif comprend un affichage électronique.

20. Dispositif selon la revendication 19, dans lequel l'affichage fournit les moyens d'indication et/ou les données d'affichage entrées dans le dispositif.

21. Dispositif selon une quelconque des revendications 7 à 20, dans lequel le dispositif comprend des moyens de transmission pour envoyer des signaux à un appareil de télétraitement des données, tel qu'un ordinateur, un serveur, un appareil Internet ou similaire.

22. Dispositif selon une quelconque des revendications 7 à 21, dans lequel le dispositif comprend des moyens de réception utilisables pour recevoir des signaux, par exemple des signaux envoyés depuis un ordinateur, un serveur ou des moyens du même type.

23. Dispositif selon la revendication 22, dans lequel le dispositif comprend des moyens de traitement pour traiter les signaux reçus.

24. Dispositif selon n'importe quelle revendication précédente, dans lequel, en utilisation, le dispositif peut être positionné sur le poussoir d'une seringue utilisée pour administrer le matériau.

25. Dispositif selon une quelconque des revendications 1 à 23, dans lequel, en utilisation, le dispositif peut être positionné sur une conduite d'alimentation en matériau, telle qu'une conduite d'injection intraveineuse.

26. Dispositif selon n'importe quelle revendication précédente, dans lequel le corps comprend une première partie fixée de façon mobile à une deuxième partie avec les moyens de verrouillage utilisables pour fixer les parties autour de l'appareil dans le premier état.

27. Dispositif selon la revendication 26, dans lequel les parties sont fixées de façon pivotante.

28. Dispositif selon la revendication 26 ou 27, dans lequel les moyens de verrouillage servent à serrer les parties autour de l'appareil une fois dans le premier état.

29. Dispositif selon la revendication 24, dans lequel le dispositif bloque ou restreint le mouvement du poussoir dans la seringue, une fois dans le premier état sur la seringue, afin d'empêcher ainsi l'administration de matériau dans la seringue.

30. Dispositif selon une quelconque des revendications 7 à 29, dans lequel le dispositif comprend une unité de télécommande, telle qu'un ordinateur, un ordinateur de poche, un téléphone, un téléphone cellulaire, un téléavertisseur ou un dispositif similaire permettant la télécommande du corps.

31. Ensemble d'administration de matériau à un corps, comprenant un appareil d'administration et un dispositif selon une quelconque des revendications précédentes.
